# EUROPEAN PATENT SPECIFICATION

(11) **EP 2 311 534 B1**
(45) Date of publication and mention of the grant of the patent: **29.04.2015**
(21) Application number: 10187363.6
(22) Date of filing: 13.10.2010
(51) Int. Cl.: A63B 33/00

(54) **Swimming Goggles**
Schwimmbrille
Lunettes de natation

(30) Priority: 19.10.2009 JP 2009240451
(43) Date of publication of application: 20.04.2011
(73) Proprietor: Yamamoto Kogaku Co., Ltd., Osaka (JP)
(72) Inventor: Tominaga, Hirofumi, Osaka (JP); Hadehara, Shinichi, Osaka (JP)
(74) Representative: Koepe & Partner Patentanwälte

(56) References cited:
- CA-A1- 2 123 375
- US-B1- 6 321 390

## Description

### BACKGROUND OF THE INVENTION

### Field of the Invention

The present invention relates to swimming goggles including goggle bodies of eyecup shape or the like, and ring-shaped face-contact pads mounted to the goggle bodies.

### Description of the Related Art

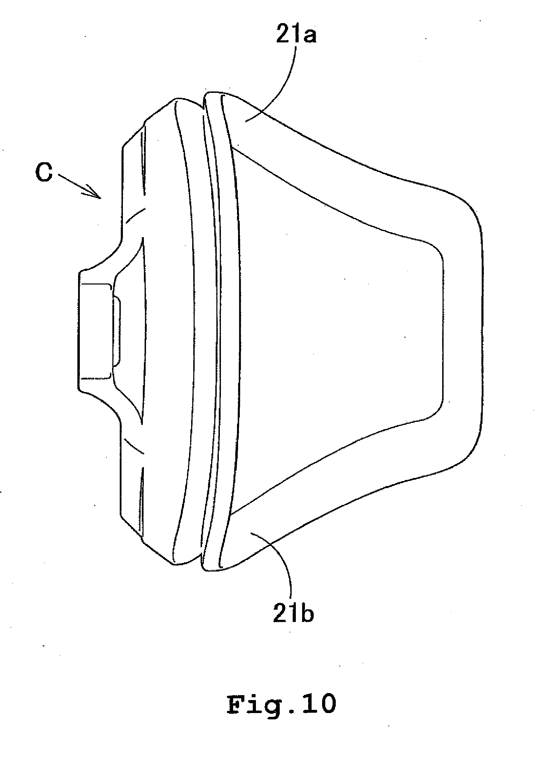

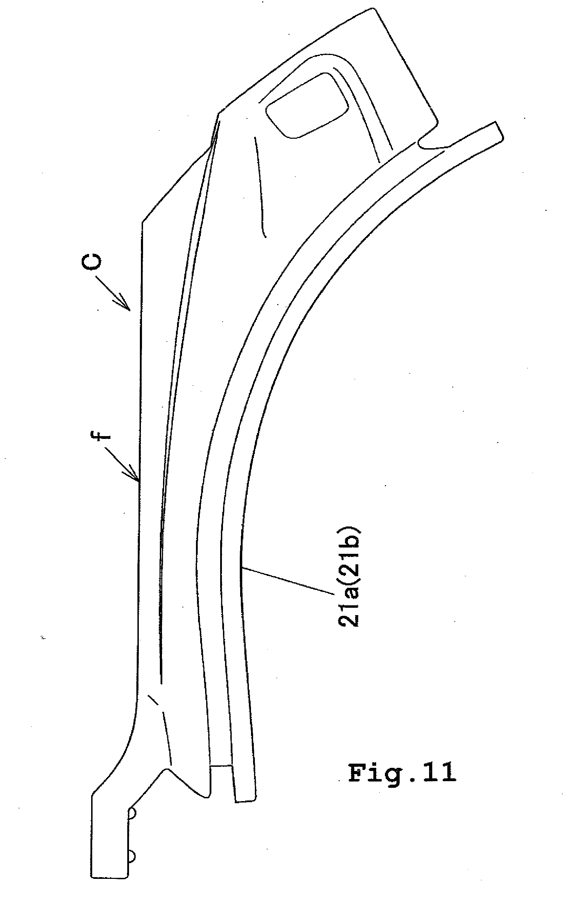

Examples of these types of conventional swimming goggles are disclosed in Patent Literatures 1 (Japanese Utility Model Laid-Open No. 06-48715) and 2 (Japanese Patent Laid-Open No. 2007-143762) as shown in Figures 10 to 14.

As shown in Figures 10 and 11, in an eyecup C of the swimming goggles disclosed in Patent Literature 1 and Patent Literature 2, an upper bottom surface 21a in contact with an upper mounting surface of a face-contact pad P mounted to the eyecup C, and a lower bottom surface 21b in contact with a lower mounting surface of the face-contact pad P are symmetrically formed. A distance from a lens front surface f to the upper bottom surface 21 a of the eyecup C is the same as a distance from the lens front surface f to the lower bottom surface 21b of the eyecup C.

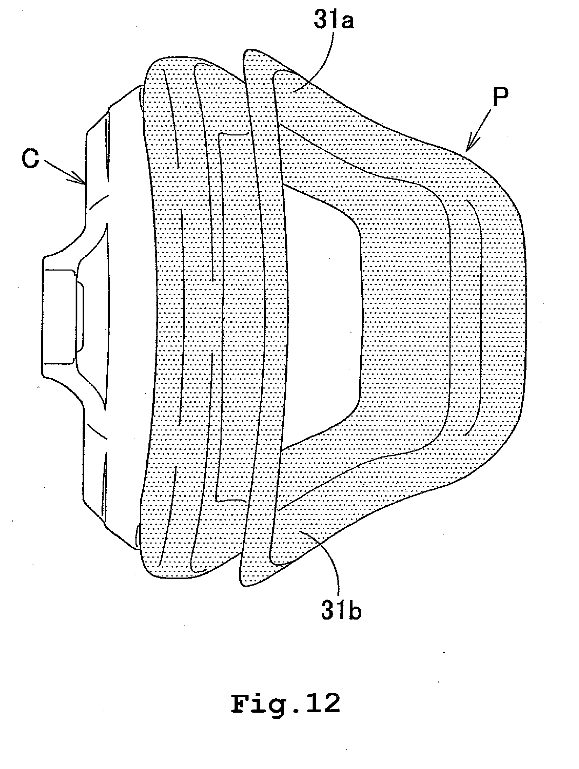

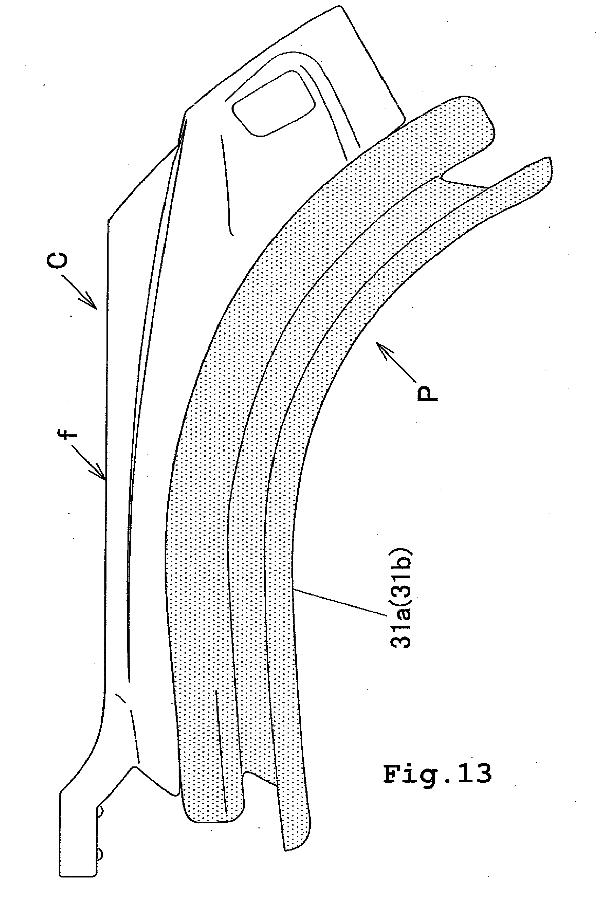

As shown in Figures 12 and 13, in the face-contact pad P mounted to the eyecup C, an upper bottom surface 31a in contact with a position above each eye of a wearer, and a lower bottom surface 31b in contact with a position below the eye of the wearer are symmetrically formed. A distance from the lens front surface f of the the eyecup C to the upper bottom surface 31 a is the same as a distance from the lens front surface f of the eyecup C to the lower bottom surface 31b.

In the conventional swimming goggles, the upper bottom surface 31a of the face-contact pad P contacts the position above each eye of a wearer, that is, a portion between the eyebrow and the upper lid, and the lower bottom surface 31b of the face-contact pad P contacts the position below the eye of the wearer, that is, a portion slightly below the lower lid. The portion between the eyebrow and the upper lid protrudes frontward from the portion slightly below the lower lid, and has a harder fitting and is thus less depressed when pushed as compared to the portion slightly below the lower lid.

Thus, when the wearer wears the conventional swimming goggles, a contact pressure at the portion between the eyebrow and the upper lid is larger than that at the portion slightly below the lower lid, and the portion between the eyebrow and the upper lid is strongly pressed by the upper bottom surface 31 a of the face-contact pad P. Thus, there is a problem that the mark of the face-contact pad P clearly remains locally in the portion, or the wearer feels uncomfortable when wearing the goggles.

Furthermore, in the conventional swimming goggles, the portion between the eyebrow and the upper lid is strongly pressed by the upper bottom surface 31a of the face-contact pad P as described above. A pressing force on the portion slightly below the lower lid by the lower bottom surface 31b of the face-contact pad P is thereby correspondingly reduced. Thus, when a high water pressure is applied at the time of diving or the like, there occurs a problem that water may leak in the goggles from the portion.

Other related swimming goggles are disclosed in United States Patent (US 6,321,390 B1). These goggles, comprise lens frames to accommodate lenses, a nose bridge to connect the lens frames, and a protective pad that is glued to the lens frames, wherein the thickness of the protective pad is inconsistent, and the side of the protective pad in contact with the user's face is designed to have different planes to fit the rims of the user's eye sockets, and specifically, the protective pad is formed in inconsistent thickness, based on the common properties at the rims of people's eye sockets, including the different depressions of upper and lower eye sockets and the depression near the nose bridge, the one side of protective pad in contact with the user's face is inclined along the upper and lower eye sockets, to make up for the difference of depressions to match the inclines, so the user can enjoy better and more comfortable contact, preventing water from seeping in even when the user is twitching his or her face.

### SUMMARY OF THE INVENTION

Accordingly, the present invention, a defined by claim 1, has been made to solve the aforementioned conventional problems, and it is an object of the invention to provide swimming goggles where a clear mark of a face-contact pad does not remain locally, a wearer does not feel uncomfortable when wearing the goggles, and water does not leak in the goggles even when a high water pressure is applied at the time of diving or the like.

To this end, in swimming goggles according to the present invention, an upper bottom surface in contact with an upper mounting surface of a face-contact pad mounted to a goggle body, and a lower bottom surface in contact with a lower mounting surface of the face-contact pad are asymmetrically formed, and a distance from a lens front surface of the goggle body and an extension surface of the lens front surface to the lower bottom surface is greater than a distance from the lens front surface of the goggle body and the extension surface of the lens front surface to the upper bottom surface.

Also, in swimming goggles according to the present invention, an upper bottom surface and a lower bottom surface of a face-contact pad mounted to a goggle body, the upper bottom surface and the lower bottom surface respectively being in contact with positions above and below each eye of a wearer, are asymmetrically formed, and a distance from a lens front surface of the goggle body and an extension surface of the lens front surface to the lower bottom surface is greater than a distance from the lens front surface of the goggle body and the extension surface of the lens front surface to the upper bottom surface.

In the swimming goggles according to the present invention, an average value of a difference S1 to a difference S20 between the distance from the lens front surface of the goggle body and the extension surface of the lens front surface to the upper bottom surface of the goggle body, and the distance from the lens front surface of the goggle body and the extension surface of the lens front surface to the lower bottom surface of the goggle body at respective measurement positions Ma to Mt provided sequentially at equal intervals in a direction from an inner corner to an outer corner of the eye of the wearer in the lens front surface of the goggle body and the extension surface of the lens front surface is set to 3.00 to 3.50 mm.

Furthermore, in the swimming goggles according to the present invention, an average value of a difference S21 to a difference S40 between the distance from the lens front surface of the goggle body and the extension surface of the lens front surface to the upper bottom surface of the face-contact pad, and the distance from the lens front surface of the goggle body and the extension surface of the lens front surface to the lower bottom surface of the face-contact pad at respective measurement positions Ma to Mt provided sequentially at equal intervals in the direction from the inner corner to the outer corner of the eye of the wearer in the lens front surface of the goggle body and the extension surface of the lens front surface is set to 3.00 to 3.50 mm.

Also, in the swimming goggles according to the present invention, a value increases gradually from the difference S1 to the difference S6. A largest value is obtained at the difference S7 or the difference S8. A value decreases gradually from the difference S9 to the difference S20.

Furthermore, in the swimming goggles according to the present invention, an average value of the difference S1 to the difference S6 is set to 2.40 to 2.80 mm. The difference S7 or the difference S8 is set to 4.60 to 5.00 mm. An average value of the difference S9 to the difference S20 is set to 3.10 to 3.50 mm.

Also, in the swimming goggles according to the present invention, a value increases gradually from the difference S21 to the difference S26. A largest value is obtained at the difference S27 or the difference S28. A value decreases gradually from the difference S29 to the difference S40.

Furthermore, in the swimming goggles according to the present invention, an average value of the difference S21 to the difference S26 is set to 2.40 to 2.80 mm. The difference S27 or the difference S28 is set to 4.60 to 5.00 mm. An average value of the difference S29 to the difference S40 is set to 3.10 to 3.50 mm.

Since the swimming goggles according to the present invention are configured as described above, a clear mark of the face-contact pad does not remain locally, a wearer does not feel uncomfortable when wearing the goggles, and water does not leak in the goggles even when a high water pressure is applied at the time of diving or the like.

### BRIEF DESCRIPTION OF THE DRAWINGS

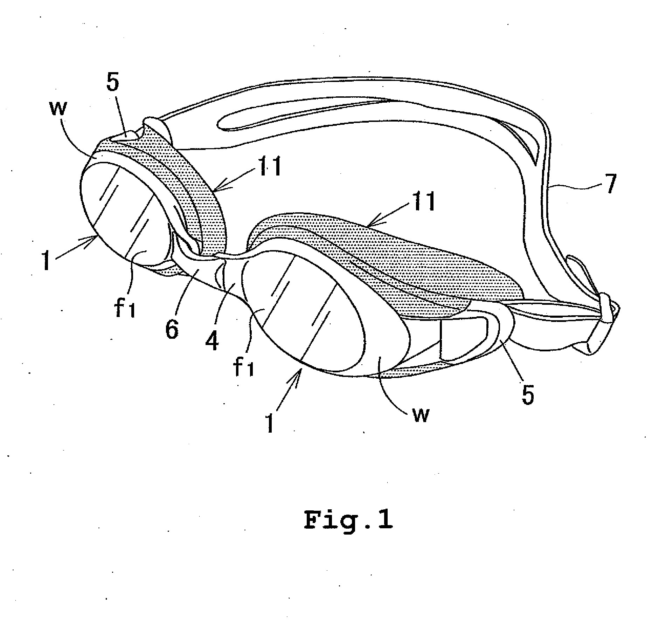

Figure 1 is a perspective view of swimming goggles according to the present invention as viewed from the front side.

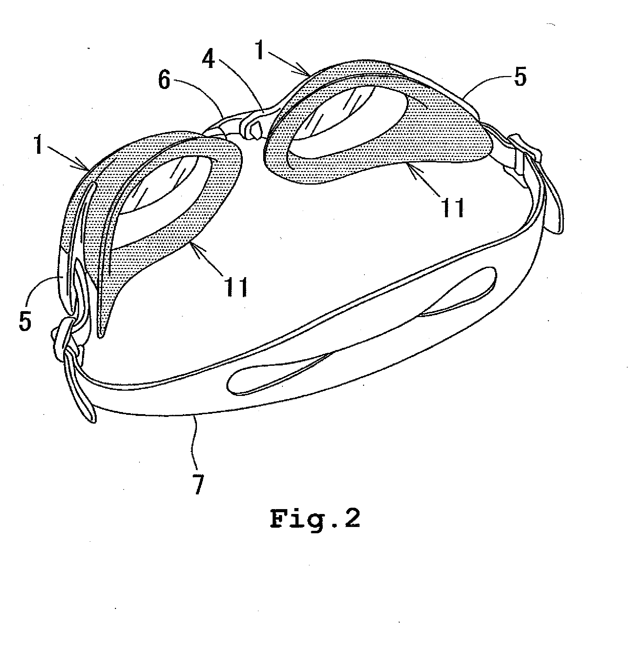

Figure 2 is a perspective view of the swimming goggles according to the present invention as viewed from the rear side.
Figure 3 is a side view of a goggle body of the swimming goggles according to the present invention to which a face-contact pad is not mounted.
Figure 4 is a plan view of the goggle body of the swimming goggles according to the present invention to which the face-contact pad is not mounted.
Figure 5 is an explanatory view illustrating a distance between an upper bottom surface and a lower bottom surface of the goggle body of the swimming goggles according to the present invention.
Figure 6 is a side view of the goggle body of the swimming goggles according to the present invention to which the face-contact pad is mounted.
Figure 7 is a plan view of the goggle body of the swimming goggles according to the present invention to which the face-contact pad is mounted.
Figure 8 is an explanatory view illustrating a distance between an upper bottom surface and a lower bottom surface of the face-contact pad of the swimming goggles according to the present invention.
Figure 9 is an explanatory view illustrating the distribution of a contact pressure of the face-contact pad of the swimming goggles according to the present invention on a portion around each eye of a wearer.
Figure 10 is a side view of an eyecup of conventional swimming goggles to which a face-contact pad is not mounted.
Figure 11 is a plan view of the eyecup of the conventional swimming goggles to which the face-contact pad is not mounted.
Figure 12 is a side view of the eyecup of the conventional swimming goggles to which the face-contact pad is mounted.
Figure 13 is a plan view of the eyecup of the conventional swimming goggles to which the face-contact pad is mounted.
Figure 14 is an explanatory view illustrating the distribution of a contact pressure of the face-contact pad of the conventional swimming goggles on a portion around each eye of a wearer.

### DETAILED DESCRIPTION OF THE PREFERRED EMBODIMENTS

In the following, an embodiment for carrying out swimming goggles according to the present invention will be described in detail based on the drawings.

The swimming goggles according to the present invention include a pair of left and right goggle bodies 1 of eyecup shape or the like made of hard synthetic resin such as transparent or semi-transparent polycarbonate. Each of the goggle bodies 1 has a lens front surface f1, and a peripheral wall portion w extending backward from the rim of the lens front surface f1. A peripheral groove 2 is provided toward the inner side on the rear end side of the peripheral wall portion w, and a mounting flange 3 is provided so as to form a rear surface of the peripheral groove 2.

A bracket 4 is provided protruding from the peripheral wall portion w at an inner end portion of each of the goggle bodies 1. A belt mounting portion 5 is also provided protruding outward from the peripheral wall portion w at an outer end portion of each of the goggle bodies 1.

A flexible coupling body 6 is engaged with the brackets 4 to thereby couple the pair of goggle bodies 1. Furthermore, opposite end portions of a stretchable fastening band 7 are respectively coupled to the belt mounting portions 5 such that the length can be adjusted.

A face-contact pad 11 made of soft synthetic resin such as elastic elastomer is mounted to the mounting flange 3 of each of the goggle bodies 1. The face-contact pad 11 includes a ring-shaped frame body 12 fitted onto the mounting flange 3, and a face-contact portion 14 formed integrally with the frame body 12 via a cushion peripheral wall 13 from the inner edge of the frame body 12. The face-contact portion 14 has a bottom surface formed so as to contact a portion around each eye of a wearer. The face-contact pad 11 may be mounted to the goggle body 1 by fitting the frame body 12 onto the mounting flange 3, or by bonding the frame body 12 to the mounting flange 3.

In the face-contact pad 11, the cushion peripheral wall 13 is formed thin and spreading toward the open side from the inner edge of the frame body 12, and the thin spreading portion is formed wider toward the outer side such that the bottom surface of the face-contact portion 14 contacts the portion around the eye of the wearer before the face-contact pad 11 is mounted to the goggle body 1.

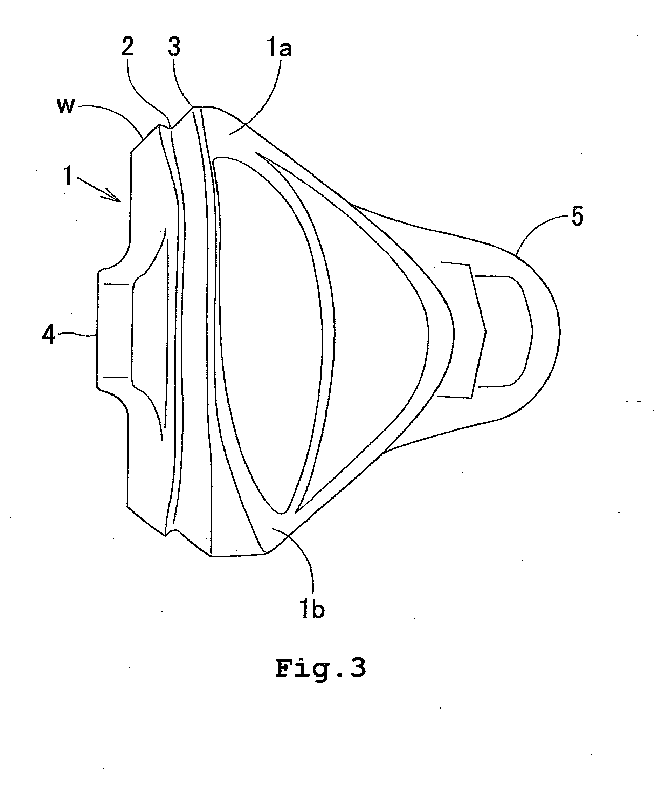

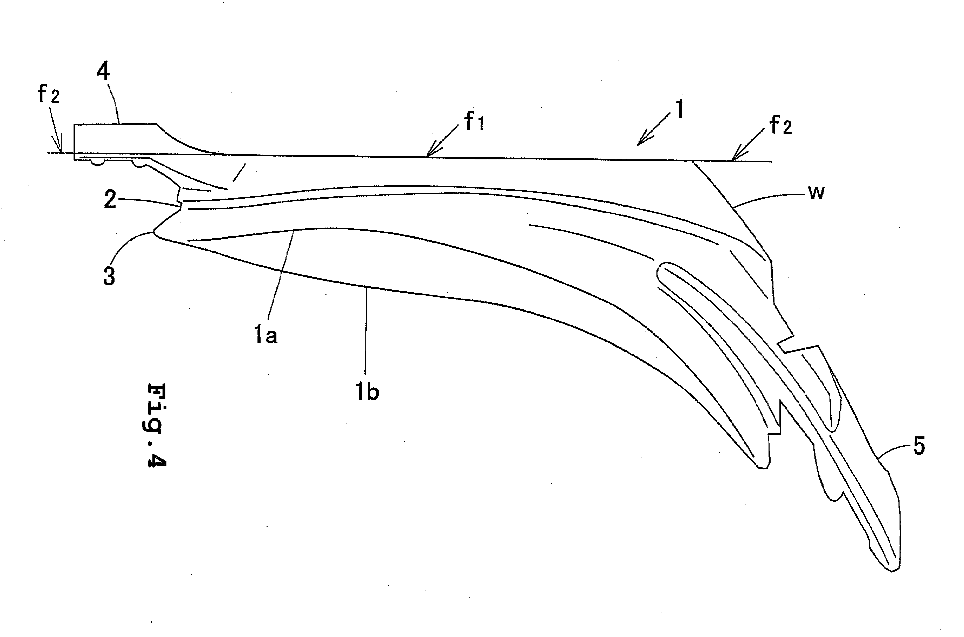

Furthermore, in the swimming goggles according to the present invention, an upper bottom surface 1 a in contact with an upper mounting surface of the face-contact pad 11 mounted to the goggle body 1, and a lower bottom surface 1b in contact with a lower mounting surface of the face-contact pad 11 are asymmetrically formed as shown in Figures 3 and 4. An entire distance from the lens front surface f1 of the goggle body 1 and an extension surface f2 of the lens front surface f1 to the lower bottom surface 1b is greater than an entire distance from the lens front surface f1 of the goggle body 1 and the extension surface f2 of the lens front surface f1 to the upper bottom surface 1a.

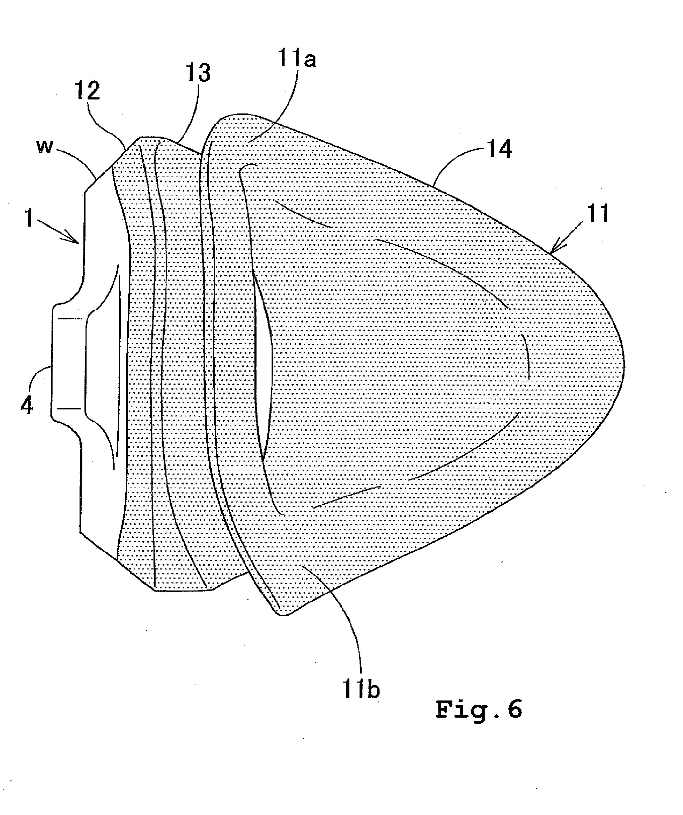

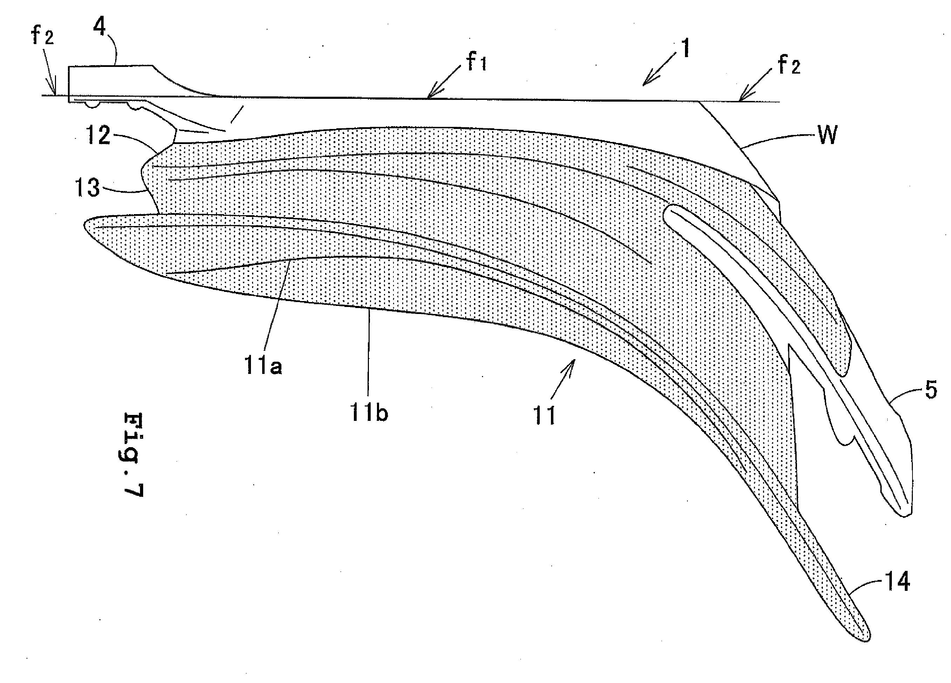

Also, in the swimming goggles according to the present invention, an upper bottom surface 11a and a lower bottom surface 11b of the face-contact pad 11 mounted to the goggle body 1, the upper bottom surface 11a and the lower bottom surface 11b respectively being in contact with positions above and below the eye of the wearer, are asymmetrically formed as shown in Figures 6 and 7. An entire distance from the lens front surface f1 of the goggle body 1 and the extension surface f2 of the lens front surface f1 to the lower bottom surface 11b is greater than an entire distance from the lens front surface f1 of the goggle body 1 and the extension surface f2 of the lens front surface f1 to the upper bottom surface 11a.

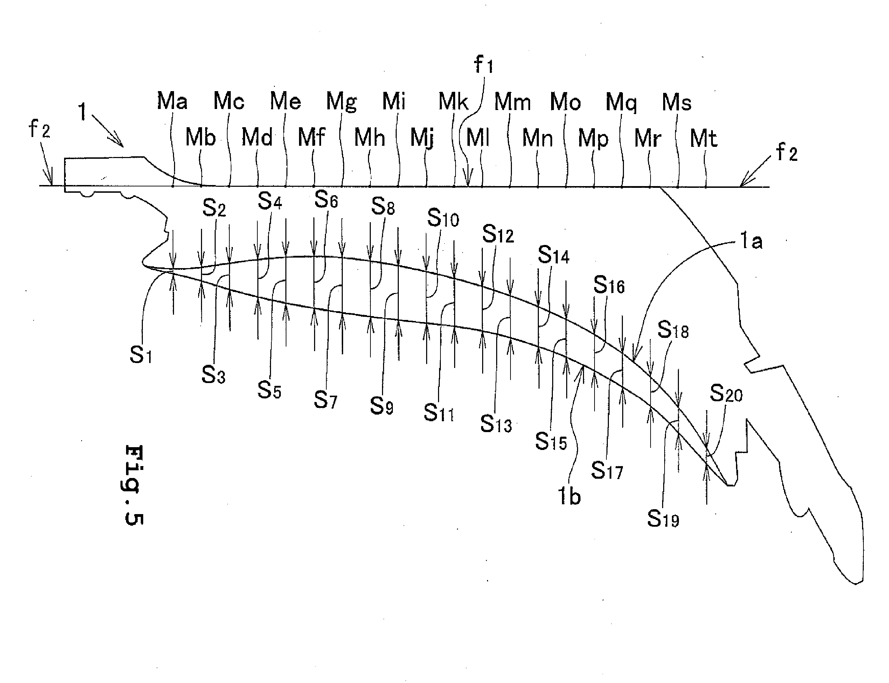

In the swimming goggles according to the present invention, differences S1 to S20 between the distance from the lens front surface f1 of the goggle body 1 and the extension surface f2 of the lens front surface f1 to the upper bottom surface 1a of the goggle body 1, and the distance from the lens front surface f1 of the goggle body 1 and the extension surface f2 of the lens front surface f1 to the lower bottom surface 1b of the goggle body 1 at respective measurement positions Ma to Mt provided sequentially at equal intervals in a direction from an inner corner to an outer corner of the eye of the wearer in the lens front surface f1 of the goggle body 1 and the extension surface f2 of the lens front surface f1 are set to 0.30 to 5.00 mm, and an average value thereof is set to 3.00 to 3.50 mm, preferably 3.25 mm as shown in Figure 5.

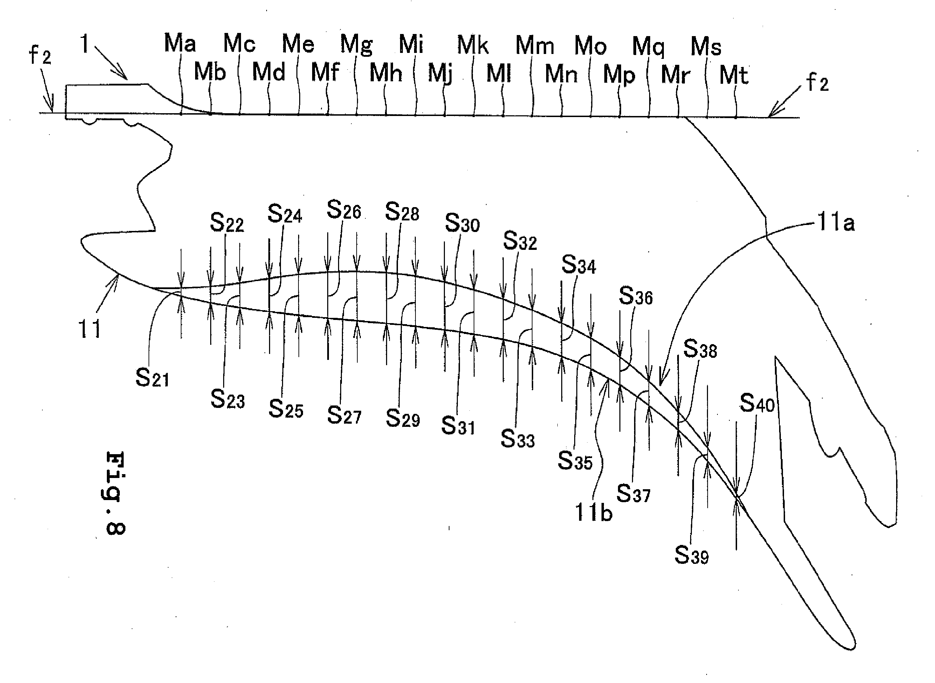

Furthermore, in the swimming goggles according to the present invention, differences S21 to S40 between the distance from the lens front surface f1 of the goggle body 1 and the extension surface f2 of the lens front surface f1 to the upper bottom surface 11a of the face-contact pad 11, and the distance from the lens front surface f1 of the goggle body 1 and the extension surface f2 of the lens front surface f1 to the lower bottom surface 11b of the face-contact pad 11 at the respective measurement positions Ma to Mt provided sequentially at equal intervals in the direction from the inner corner to the outer corner of the eye of the wearer in the lens front surface f1 of the goggle body 1 and the extension surface f2 of the lens front surface f1 are set to 0.3 0 to 5.00 mm, and an average value thereof is set to 3.00 to 3.50 mm, preferably 3.25 mm as shown in Figure 8.

The value increases gradually from the difference S1 to S6. The largest value is obtained at the difference S7 or S8. The value decreases gradually from the difference S9 to S20. An average value of the differences S1 to S6 is set to 2.40 to 2.80 mm, preferably 2.62 mm. The difference S7 or S8 is set to 4.60 to 5.00 mm, preferably 4.82 mm. An average value of the differences S9 to S20 is set to 3.10 to 3.50 mm, preferably 3.31 mm.

The value increases gradually from the difference S21 to S26. The largest value is obtained at the difference S27 or S28. The value decreases gradually from the difference S29 to S40. An average value of the differences S21 to S26 is set to 2.40 to 2.80 mm, preferably 2.62 mm. The difference S27 or S28 is set to 4.60 to 5.00 mm, preferably 4.82 mm. An average value of the differences S29 to S40 is set to 3.10 to 3.50 mm, preferably 3.31 mm.

In the swimming goggles according to the present invention, concrete figures of the differences S1 to S20 are as follows. For example, the difference S1 is 0.30 mm, the difference S2 is 1.12 mm, the difference S3 is 2.26 mm, the difference S4 is 3.29 mm, the difference S5 is 4.10 mm, the difference S6 is 4.64 mm, the difference S7 is 4.84 mm, the difference S8 is 4.80 mm, the difference S9 is 4.65 mm, the difference S10 is 4.44 mm, the difference S11 is 4.20 mm, the difference S12 is 3.97 mm, the difference S13 is 3.75 mm, the difference S 14 is 3.53 mm, the difference S 15 is 3.31 mm, the difference S16 is 3.09 mm, the difference S17 is 2.85 mm, the difference S18 is 2.56 mm, the difference S19 is 2.09 mm, and the difference S20 is 1.29 mm. The figures are set based on measurement data on the contour at a portion between the eyebrow and the upper lid and a portion slightly below the lower lid of Japanese men in their twenties by the Research Institute of Human Engineering for Quality Life.

Although concrete figures of the differences S21 to S40 are not described here, the figures corresponding to the differences S1 to S20 can be employed, but not limited thereto.

That is, by setting the differences S1 to S20 and the differences S21 to S40 to the figures as described above, the swimming goggles according to the present invention can produce desired effects that a clear mark of the face-contact pad does not remain locally, a wearer does not feel uncomfortable when wearing the goggles, and water does not leak in the goggles even when a high water pressure is applied at the time of diving or the like. Setting the differences S21 to S40 to the figures as described above can provide the similar effects, regardless of setting the differences S1 to S20.

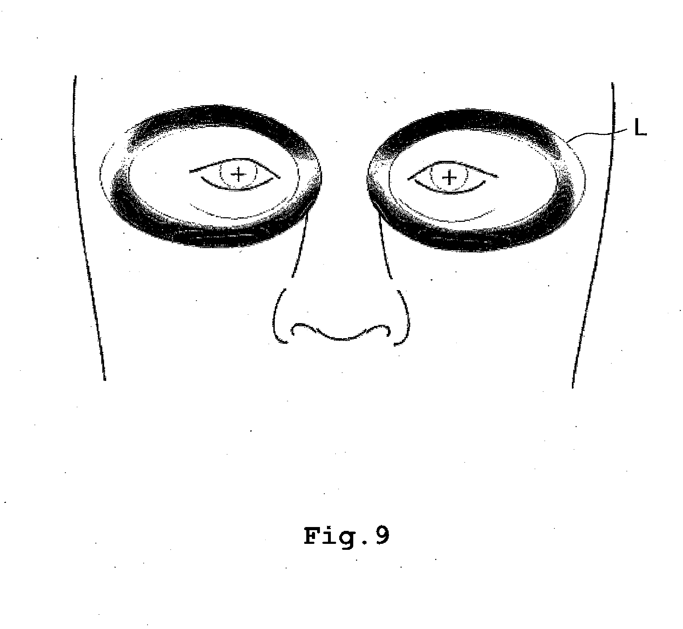

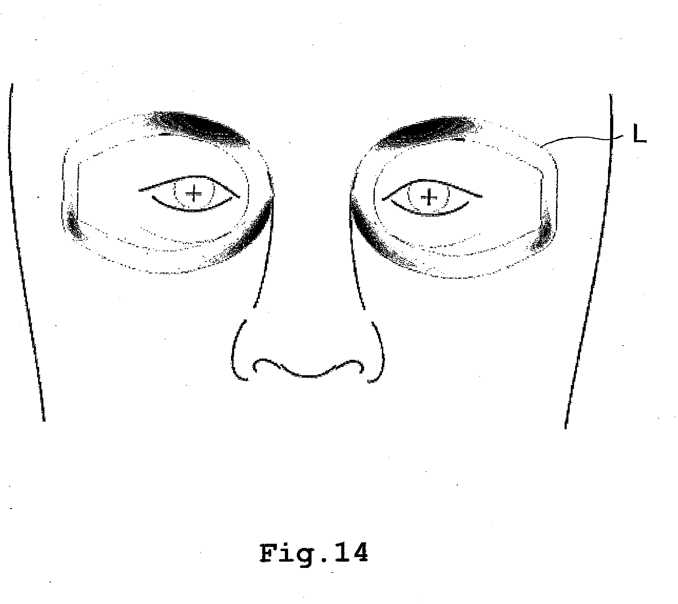

Figure 9 is an explanatory view illustrating the distribution of a contact pressure of the face-contact pad of the swimming goggles according to the present invention on a portion around each eye of a wearer. Figure 14 is an explanatory view illustrating the distribution of a contact pressure of a face-contact pad of conventional swimming goggles shown in Figures 12 and 13 on a portion around each eye of a wearer. The drawings are based on the measurement data by the Research Institute of Human Engineering for Quality Life.

An outline L in the drawings indicates a contact surface between the face-contact pad and the portion around the eye of the wearer. A color gradation of 3 to 5 stages shown within the outline L indicates the distribution of the contact pressure. A darker color represents a larger contact pressure.

According to Figure 9, in the swimming goggles according to the present invention, the entire portion between the eyebrow and the upper lid, and the entire portion slightly below the lower lid are pressed by the face-contact pad in a balanced manner. Thus, the clear mark of the face-contact pad does not remain locally, and the wearer does not feel uncomfortable when wearing the goggles.

Meanwhile, in the conventional swimming goggles, the portion between the eyebrow and the upper lid, especially a portion close to the glabella between the eyebrow and the upper lid, and a portion close to the nose slightly below the lower lid are strongly pressed by the face-contact pad as shown in Figure 14. The clear mark of the face-contact pad thereby remains locally in the portions. The wearer also feels uncomfortable when wearing the goggles.

Furthermore, according to Figure 9, in the swimming goggles according to the present invention, the entire portion between the eyebrow and the upper lid, and the entire portion slightly below the lower lid are strongly pressed by the face-contact pad. Thus, even when a high water pressure is applied at the time of diving or the like, water does not leak in the goggles.

Meanwhile, as shown in Figure 14, in the conventional swimming goggles, although the portion close to the glabella between the eyebrow and the upper lid and the portion close to the nose slightly below the lower lid are strongly pressed by the face-contact pad, a pressing force of the face-contact pad on another portion is correspondingly reduced. Thus, when a high water pressure is applied at the time of diving or the like, water may leak in the goggles from the portion.

### [Reference Signs List]

- 1: Goggle body
- 1a: Upper bottom surface
- 1b: Lower bottom surface
- 11: Face-contact pad
- 11a: Upper bottom surface
- 11b: Lower bottom surface
- f1: Lens front surface
- f2: Extension surface
- Ma to Mt: Measurement position
- S1 to S40: Difference

## Claims

1. Swimming goggles comprising:
a goggle body (1);
a face-contact pad (11) mounted to the goggle body (1) including a ring-shaped frame body (12) and a face-contact portion (14) formed integrally with the frame body (12) via a cushion peripheral wall (13) from the inner edge of the frame body (12); an upper bottom surface (11a) of the face-contact pad (11);
a lower bottom surface (11b) of the face-contact pad (11); and
wherein the upper bottom surface (11a) and the lower bottom surface (11b),respectively suitable for being in contact with positions above and below each eye of a wearer, are asymmetrically formed, a distance from a lens front surface (f1) of the goggle body (1) and an extension surface (f2) of the lens front surface (f1) to the lower bottom surface (11b) is greater than a distance from the lens front surface (f1) of the goggle body (1) and the extension surface (f2) of the lens front surface (f1) to the upper bottom surface (11a) by a difference that varies along a lateral direction, an average value of 20 individual differences between the distance from the lens front surface (f1) of the goggle body (1) and the extension surface (f2) of the lens front surface (f1) to the upper bottom surface (11a) of the face-contact pad (11), and the distance from the lens front surface (f1) of the goggle body (1) and the extension surface (f2) of the lens front surface (f1) to the lower bottom surface (11b) of the face-contact pad (11) at respective measurement positions (Ma to Mt) provided sequentially at equal intervals in a direction from an inner corner to an outer corner of the eye of the wearer in the lens front surface (f1) of the goggle body (1) and the extension surface (f2) of the lens front surface (f1) along the entire width shared by upper bottom surface (11a) and lower bottom surface (11b) both suitable for being in contact with respective positions above and below each eye of a wearer is set to 3.00 to 3.50 mm, and a value increases gradually from the first difference at a respective measurement position (Ma) to the sixth difference at a respective measurement positions (Mf), a largest value is obtained at the seventh difference at a respective measurement position (Mg) or the eighth difference at a respective measurement position (Mh), and a value decreases gradually from the ninth difference at a respective measurement position (Mi) to the twentieth difference at a respective measurement position (Mt).

2. Swimming goggles according to claim 1, which the goggle body (1) has an upper bottom surface (1a) in contact with an upper mounting surface of the face-contact pad (11) and a lower bottom surface (1b) in contact with a lower mounting surface of the face-contact pad (11), wherein the upper bottom surface (1a) and the lower bottom surface (1b) are asymmetrically formed, and a distance from a lens front surface (f1) of the goggle body (1) and an extension surface (f2) of the lens front surface (f1) to the lower bottom surface (1b) is greater than a distance from the lens front surface (f1) of the goggle body (1) and the extension surface (f2) of the lens front surface (f1) to the upper bottom surface (1a).

3. The swimming goggles according to claim 2, wherein an average value of 20 individual differences between the distance from the lens front surface (f1) of the goggle body (1) and the extension surface (f2) of the lens front surface (f1) to the upper bottom surface (1a) of the goggle body (1), and the distance from the lens front surface (f1) of the goggle body (1) and the extension surface (f2) of the lens front surface (f1) to the lower bottom surface (1b) of the goggle body (1) at respective measurement positions (Ma to Mt) provided sequentially at equal intervals in a direction from an inner corner to an outer corner of the eye of the wearer in the lens front surface (f1) of the goggle body (1) and the extension surface (f2) of the lens front surface (f1) along the entire width shared by upper bottom surface (1a) and lower bottom surface (1b) is set to 3.00 to 3.50 mm.

4. The swimming goggles according to claim 3, wherein a value increases gradually from the first difference at a respective measurement position (Ma) to the sixth difference at a respective measurement positions (Mf), a largest value is obtained at the seventh difference at a respective measurement position (Mg) or the eighth difference at a respective measurement position (Mh), and a value decreases gradually from the ninth difference at a respective measurement position (Mi) to the twentieth difference at a respective measurement position (Mt).

5. The swimming goggles according to claim 3 or 4, wherein an average value of the first difference at a respective measurement position (Ma) to the sixth difference at a respective measurement positions (Mf) is set to 2.40 to 2.80 mm, the seventh difference at a respective measurement position (Mg) or the eighth difference at a respective measurement position (Mh) is set to 4.60 to 5.00 mm, and an average value of the ninth difference at a respective measurement position (Mi) to the twentieth difference at a respective measurement position (Mt) is set to 3.10 to 3.50 mm.

6. The swimming goggles according to claim 1, wherein an average value of the first difference at a respective measurement position (Ma) to the sixth difference at a respective measurement position (Mf) is set to 2.40 to 2.80 mm, the seventh difference at a respective measurement position (Mg) or the eighth difference at a respective measurement position (Mh) is set to 4.60 to 5.00 mm, and an average value of the ninth difference at a respective measurement position (Mi) to the twentieth difference at a respective measurement position (Mt) is set to 3.10 to 3.50 mm.

## Patentansprüche

1. Schwimmbrille, umfassend
einen Brillen-Körper (1);
ein Gesichts-Kontakt-Polster (11), das an dem Brillen-Körper (1) angebracht ist und einen ringförmigen Rahmen-Körper (12) und einen Gesichts-Kontakt-Teil (14) einschließt, der integral mit dem Rahmen-Körper (12) über eine Polster-Umfangs-Wand (13) von der inneren Kante des Rahmen-Körpers (12) geformt ist;
eine obere Unterseiten-Fläche (11a) des Gesichts-Kontakt-Polsters (11);
eine untere Unterseiten-Fläche (11b) des Gesichts-Kontakt-Polsters (11); und
wobei die obere Unterseiten-Fläche (11a) und die untere Unterseiten-Fläche (11b), die jeweils geeignet sind dafür, in Kontakt mit Positionen oberhalb und unterhalb jedes Auges eines Trägers zu sein, asymmetrisch geformt sind; eine Entfernung von einer Linsen-Vorderseiten-Fläche (f1) des Brillen-Körpers (1) und einer Erstreckungs-Fläche (f2) der Linsen-Vorderseiten-Fläche (f1) zu der unteren Unterseiten-Fläche (11b) um eine Differenz, die entlang einer seitlichen Richtung variiert, größer ist als eine Entfernung von der Linsen-Vorderseiten-Fläche (f1) des Brillen-Körpers (1) und der Erstreckungs-Fläche (f2) der Linsen-Vorderseiten-Fläche (f1) zu der oberen Unterseiten-Fläche (11a); ein mittlerer Wert von 20 einzelnen Differenzen zwischen der Entfernung von der Linsen-Vorderseiten-Fläche (f1) des Brillen-Körpers (1) und einer Erstreckungs-Fläche (f2) der Linsen-Vorderseiten-Fläche (f1) zu der oberen Unterseiten-Fläche (11a) des Gesichts-Kontakt-Polsters (11) und der Entfernung von der Linsen-Vorderseiten-Fläche (f1) des Brillen-Körpers (1) und einer Erstreckungs-Fläche (f2) der Linsen-Vorderseiten-Fläche (f1) zu der unteren Unterseiten-Fläche (11b) des Gesichts-Kontakt-Polsters (11) an entsprechenden Mess-Positionen (Ma bis Mt), vorgesehen fortlaufend in gleichen Abständen in einer Richtung von einer inneren Ecke zu einer äußeren Ecke des Auges des Trägers in der Linsen-Vorderseiten-Fläche (f1) des Brillen-Körpers (1) und der Erstreckungs-Fläche (f2) der Linsen-Vorderseiten-Fläche (f1) entlang der gesamten von der oberen Unterseiten-Fläche (11a) und der unteren Unterseiten-Fläche (11b) geteilten Breite, die beide geeignet sind, um in Kontakt mit jeweiligen Positionen oberhalb und unterhalb jedes Auges eines Trägers zu sein, festgesetzt ist auf 3,00 bis 3,50 mm, und ein Wert schrittweise ansteigt von der ersten Differenz an einer jeweiligen Mess-Position (Ma) bis zu der sechsten Differenz an einer jeweiligen Mess-Position (Mf), ein größter Wert erhalten wird an der siebten Differenz an einer jeweiligen Mess-Position (Mg) oder der achten Differenz an einer jeweiligen Mess-Position (Mh), und ein Wert schrittweise sinkt von der neunten Differenz an einer jeweiligen Mess-Position (Mi) bis zu der zwanzigsten Differenz an einer jeweiligen Mess-Position (Mt).

2. Schwimmbrille nach Anspruch 1, bei der der Brillen-Körper (1) eine obere Unterseiten-Fläche (1a), die in Kontakt mit einer oberen Montage-Fläche des Gesichts-Kontakt-Polsters (11) ist, und eine untere Unterseiten-Fläche (1b) aufweist, die in Kontakt mit einer unteren Montage-Fläche des Gesichts-Kontakt-Polsters (11) ist, wobei die obere Unterseiten-Fläche (1a) und die untere Unterseiten-Fläche (1b) asymmetrisch geformt sind, und eine Entfernung von einer Linsen-Vorderseiten-Fläche (f1) des Brillen-Körpers (1) und einer Erstreckungs-Fläche (f2) der Linsen-Vorderseiten-Fläche (f1) zu der unteren Unterseiten-Fläche (1b) größer ist als eine Entfernung von der Linsen-Vorderseiten-Fläche (f1) des Brillen-Körpers (1) und der Erstreckungs-Fläche (f2) der Linsen-Vorderseiten-Fläche (f1) zu der oberen Unterseiten-Fläche (1a).

3. Schwimmbrille nach Anspruch 2, wobei ein mittlerer Wert von 20 einzelnen Differenzen zwischen der Entfernung von der Linsen-Vorderseiten-Fläche (f1) des Brillen-Körpers (1) und der Erstreckungs-Fläche (f2) der Linsen-Vorderseiten-Fläche (f1) zu der oberen Unterseiten-Fläche (1a) des Brillen-Körpers (1) und der Entfernung von der Linsen-Vorderseiten-Fläche (f1) des Brillen-Körpers (1) und einer Erstreckungs-Fläche (f2) der Linsen-Vorderseiten-Fläche (f1) zu der unteren Unterseiten-Fläche (1b) des Brillen-Körpers (1) an entsprechenden Mess-Positionen (Ma bis Mt), vorgesehen fortlaufend in gleichen Abständen in einer Richtung von einer inneren Ecke zu einer äußeren Ecke des Auges des Trägers in der Linsen-Vorderseiten-Fläche (f1) des Brillen-Körpers (1) und der Erstreckungs-Fläche (f2) der Linsen-Vorderseiten-Fläche (f1) entlang der gesamten von der oberen Unterseiten-Fläche (1a) und der unteren Unterseiten-Fläche (1b) geteilten Breite, festgesetzt ist auf 3,00 bis 3,50 mm.

4. Schwimmbrille nach Anspruch 3, wobei ein Wert schrittweise ansteigt von der ersten Differenz an einer jeweiligen Mess-Position (Ma) bis zu der sechsten Differenz an einer jeweiligen Mess-Position (Mf), ein größter Wert erhalten wird an der siebten Differenz an einer jeweiligen Mess-Position (Mg) oder der achten Differenz an einer jeweiligen Mess-Position (Mh), und ein Wert schrittweise sinkt von der neunten Differenz an einer jeweiligen Mess-Position (Mi) bis zu der zwanzigsten Differenz an einer jeweiligen Mess-Position (Mt).

5. Schwimmbrille nach Anspruch 3 oder 4, wobei ein mittlerer Wert der ersten Differenz an einer jeweiligen Mess-Position (Ma) bis zu der sechsten Differenz an einer jeweiligen Mess-Position (Mf) festgesetzt wird auf 2,40 bis 2,80 mm; die siebte Differenz an einer jeweiligen Mess-Position (Mg) oder die achte Differenz an einer jeweiligen Mess-Position (Mh) festgesetzt wird auf 4,60 bis 5,00 mm; und ein mittlerer Wert der neunten Differenz an einer jeweiligen Mess-Position (Mi) bis zu der zwanzigsten Differenz an einer jeweiligen Mess-Position (Mt) festgesetzt wird auf 3,10 bis 3,50 mm.

6. Schwimmbrille nach Anspruch 1, wobei ein mittlerer Wert der ersten Differenz an einer jeweiligen Mess-Position (Ma) bis zu der sechsten Differenz an einer jeweiligen Mess-Position (Mf) festgesetzt wird auf 2,40 bis 2,80 mm; die siebte Differenz an einer jeweiligen Mess-Position (Mg) oder die achte Differenz an einer jeweiligen Mess-Position (Mh) festgesetzt wird auf 4,60 bis 5,00 mm; und ein mittlerer Wert der neunten Differenz an einer jeweiligen Mess-Position (Mi) bis zu der zwanzigsten Differenz an einer jeweiligen Mess-Position (Mt) festgesetzt wird auf 3,10 bis 3,50 mm.

## Revendications

1. Lunettes de natation comprenant :
- un corps de lunettes (1) ;
- un tampon de contact-visage (11) monté sur le corps de lunettes (1) comprenant un corps de châssis en forme d'anneau (12) et une partie de contact-visage (14) formée intégralement avec le corps de châssis (12) via une paroi périphérique amortisseur (13) depuis le bord intérieur du corps de châssis (12),
- une surface de fond supérieure (11a) du tampon de contact-visage (11) ;
- une surface de fond inférieure (11b) du tampon de contact-visage (11) ; et
dans lesquelles la surface de fond supérieure (11a) et la surface de fond inférieure (11 b), respectivement adaptées pour être en contact avec des positions au-dessus et au-dessous de chaque oeil du porteur des lunettes, sont formées de manière asymétrique, une distance d'une surface frontale de lentille (f1) du corps de lunettes (1) et d'une surface d'extension (f2) de la surface frontale de lentille (f1) jusqu'à la surface de fond inférieure (11 b) est supérieure à une distance de la surface frontale de lentille (f1) du corps de lunettes (1) et de la surface d'extension (f2) de la surface de lentille frontale (f1) jusqu'à la surface de fond supérieure (11a) d'une différence qui varie le long d'une direction latérale, une valeur moyenne de 20 différences individuelles entre la distance depuis la surface frontale de lentille (f1) du corps de lunettes (1) et de la surface d'extension (f2) de la surface frontale de lentille (f1) jusqu'à la surface de fond supérieure (11a) du tampon de contact-visage (11), et la distance depuis la surface frontale de lentille (f1) du corps de lunettes (1) et de la surface d'extension (f2) de la surface frontale de lentille (f1) jusqu'à la surface de fond inférieure (11 b) du tampon de contact-visage (11) à des positions de mesure respectives (Ma à Mt) fournies séquentiellement à des intervalles égaux dans une direction depuis un coin intérieur jusqu'à un coin extérieur de l'oeil du porteur des lunettes dans la surface frontale de lentille (f1) du corps de lunettes (1) et de la surface d'extension (f2) de la surface frontale de lentille (f1) le long de la largeur entière partagée par la surface de fond supérieure (11a) et la surface de fond inférieure (11 b) les deux étant appropriées pour être en contact avec des positions au-dessus et au-dessous de chaque oeil d'un porteur des lunettes est établie entre 3,00 et 3,50 mm, et une valeur augmente graduellement depuis la première différence à une position de mesure respective (Ma) jusqu'à la sixième différence à une position de mesure respective (Mf), une valeur la plus grande est obtenue à la septième différence à une position de mesure respective (Mg) à la huitième différence à une position de mesure respective (Mh), et une valeur diminue graduellement depuis la neuvième différence à une position de mesure respective (Mi) jusqu'à la vingtième différence à une position de mesure respective (Mt).

2. Lunettes de natation selon la revendication 1, dans lesquelles le corps de lunettes (1) présente une surface de fond supérieure (1a) en contact avec une surface de montage supérieure du tampon de contact-visage (11) et une surface de fond inférieure (1 b) en contact avec un surface de montage inférieure du tampon de contact-visage (11), la surface de fond supérieure (1a) et la surface de fond inférieure (1b) étant formées de manière asymétrique, et une distance à partir d'une surface frontale de lentille (f1) du corps de lunettes (1) et d'une surface d'extension (f2) de la surface frontale de lentille (f1) jusqu'à la surface de fond inférieure (1 b) est supérieure à une distance de la surface frontale de lentille (f1) du corps de lunettes (1) et de la surface d'extension (f2) de la surface de lentille frontale (f1) jusqu'à la surface de fond supérieure (1 a).

3. Lunettes de natation selon la revendication 2, dans lesquelles une valeur moyenne de 20 différences individuelles entre la distance depuis la surface frontale de lentille (f1) du corps de lunettes (1) et de la surface d'extension (f2) de la surface frontale de lentille (f1) jusqu'à la surface de fond supérieure (1a) du corps de lunettes (1), et la distance depuis la surface frontale de lentille (f1) du corps de lunettes (1) et de la surface d'extension (f2) de la surface frontale de lentille (f1) jusqu'à la surface de fond inférieure (1b) du corps de lunettes (1) à des positions de mesure respectives (Ma à Mt) fournies séquentiellement à des intervalles égaux dans une direction depuis un coin intérieur jusqu'à un coin extérieur de l'oeil du porteur des lunettes dans la surface frontale de lentille (f1) du corps de lunettes (1) et de la surface d'extension (f2) de la surface frontale de lentille (f1) le long de la largeur entière partagée par la surface de fond supérieure (1a) et la surface de fond inférieure (1 b) est établie entre 3,00 et 3,50 mm.

4. Lunettes de natation selon la revendication 3, dans lesquelles une valeur augmente graduellement depuis la première différence à une position de mesure respective (Ma) jusqu'à la sixième différence à une position de mesure respective (Mf), une valeur la plus grande est obtenue à la septième différence à une position de mesure respective (Mg) ou à la huitième différence à une position de mesure respective (Mh), et une valeur diminue graduellement depuis la neuvième différence à une position de mesure respective (Mi) jusqu'à la vingtième différence à une position de mesure respective (Mt).

5. Lunettes de natation selon la revendication 3 ou 4, dans lesquelles une valeur moyenne de la première différence à une position de mesure respective (Ma) jusqu'à la sixième différence à une position de mesure respective (Mf) est établie entre 2,40 et 2,80 mm, la septième différence à une position de mesure respective (Mg) ou la huitième différence à une position de mesure respective (Mh) est établie entre 4,60 et 5,00 mm et une valeur moyenne de la neuvième différence à une position de mesure respective (Mi) jusqu'à la vingtième différence à une position de mesure respective (Mt) est établie entre 3,10 et 3,50 mm.

6. Lunettes de natation selon la revendication 1 dans lesquelles une valeur moyenne de la première différence à une position de mesure respective (Ma) jusqu'à la sixième différence à une position de mesure respective (Mf) est établie entre 2,40 et 2,80 mm, la septième différence à une position de mesure respective (Mg) ou la huitième différence à une position de mesure respective (Mh) est établie entre 4,60 et 5,00 mm et une valeur moyenne de la neuvième différence à une position de mesure respective (Mi) jusqu'à la vingtième différence à une position de mesure respective (Mt) est établie entre 3,10 et 3,50 mm.
